Europäisches Patentamt

European Patent Office

Office européen des brevets

(11) Veröffentlichungsnummer: **0 321 679 B1**

# EUROPÄISCHE PATENTSCHRIFT

(45) Veröffentlichungstag der Patentschrift: **03.06.92**

(51) Int. Cl.⁵: **B26B 21/44**, A61K 7/15

(21) Anmeldenummer: **88117974.1**

(22) Anmeldetag: **28.10.88**

(54) **Rasierapparat und Verfahren zur Herstellung einer Fläche geringen Reibungswiderstands an einem Rasierapparat.**

(30) Priorität: **19.12.87 DE 3743298**

(43) Veröffentlichungstag der Anmeldung:
**28.06.89 Patentblatt 89/26**

(45) Bekanntmachung des Hinweises auf die Patenterteilung:
**03.06.92 Patentblatt 92/23**

(84) Benannte Vertragsstaaten:
**AT BE CH DE ES FR GB GR IT LI LU NL SE**

(56) Entgegenhaltungen:
**EP-A- 0 184 440**
**GB-A- 2 009 017**
**GB-A- 2 024 082**
**US-A- 3 715 942**
**US-A- 3 903 410**

(73) Patentinhaber: **Wilkinson Sword Gesellschaft mit beschränkter Haftung**
**Schützenstrasse 110**
**W-5650 Solingen 1(DE)**

(72) Erfinder: **Althaus, Wolfgang**
**Hülsberg 94**
**W-5600 Wuppertal(DE)**
Erfinder: **Thöne, Jochen**
**Erbschlöherstrasse 67**
**W-5600 Wuppertal 21(DE)**
Erfinder: **Lorenz, Donald H.**
**12 Radel Place**
**Basking Ridge, NJ 07920(US)**
Erfinder: **Creasy, Walter S.**
**272 White Oak Ridge**
**Bridgewater, NJ 08807(US)**

(74) Vertreter: **Patentanwälte Dipl.-Ing. Alex Stenger Dipl.-Ing. Wolfram Watzke Dipl.-Ing. Heinz J. Ring**
**Kaiser-Friedrich-Ring 70**
**W-4000 Düsseldorf 11(DE)**

## Beschreibung

Die Erfindung betrifft einen Rasierapparat mit einem Träger für mindestens eine Rasierklinge, bei dem eine die Haut des Benutzers während des Rasiervorgangs berührende Fläche mit einem Gleitmittel versehen ist sowie ein Verfahren zur Herstellung einer solchen Fläche geringen Reibungswiderstands an einem Rasierapparat.

Zur Naßrasur sind verschiedene Rasierhilfsmittel bekannt, die die Aufgabe erfüllen sollen, den Reibungswiderstand zwischen der Haut und dem Rasierapparat während des Rasiervorgangs zu verringern, nämlich Rasierschaum, Rasierseife, die Barthaare erweichende Mittel sowie medizinische oder kosmetische Wirkstoffe oder Kombinationen all dieser. Derartige Rasierhilfsmittel reduzieren entweder die Schneidarbeit, die zur Durchtrennung der Barthaare aufgewandt werden muß, durch Erweichen der Barthaare oder wirken als Gleitmittel, das die Reibkraft zwischen den die Haut berührenden Kunststoffteilen des Rasierapparats und der Haut des Benutzers reduziert.

Ein neuerer Vorschlag (siehe z.B. EP-A-0 184 440) besteht darin, Polyäthylenoxid als integrales, wasserlösliches Rasierhilfsmittel auf einem Rasierapparat zu benutzen oder einen spritzgegossenen Streifen aus einem hydrophoben Polymer, wie Polystyrol, und einem hydrophilen Polymer, wie Polyäthylenoxid, in einer Ausnehmung auf der Kappe des Rasierapparats anzubringen. In beiden Fällen wird angestrebt, die wasserlösliche Polymerkomponente kontinuierlich im Verlaufe der Naßrasur, bedingt durch die Anwesenheit von Wasser auf der zu rasierenden Hautfläche herauszulösen und auf der Haut einen Gleitfilm zu erzeugen. Dieser reduziert die bei der Rasur auftretenden Reibungskräfte und gestaltet den Rasiervorgang weicher und komfortabler.

Bei der Verwendung derartiger Rasierhilfsmittel ergeben sich mehr oder weniger gravierende Nachteile dadurch, daß diese im wesentlichen aus einem wasserlöslichen, polymeren Material bestehen, das sich während des Rasiervorgangs herauslöst und auf der Haut des Benutzers einen Film bildet, der auf den bereits rasierten Hautflächen antrocknet. Es wird somit erforderlich, das angetrocknete Gleitmittel nach der Rasur von der Haut abzuwaschen, was in Abhängigkeit vom Härtegrad des zur Verfügung stehenden Wassers schwierig sein kann und beim Benutzer unter Umständen ein klebriges Gefühl hinterläßt. Es ist somit nicht auszuschließen, daß Reste des herausgelösten Gleitmittels auf der Haut verbleiben und bei wiederholter Anwendung und empfindlicher Haut die Gefahr von Hautirritationen verursachen können. Darüber hinaus geht der Vorgang der Herauslösung des Gleitmittels während des Rasiervorgangs nachteiligerweise unkontrolliert vor sich. Beispielsweise in Abhängigkeit von der Temperatur des benutzten Wassers, der Länge der Zeit, die der Rasierapparat im Wasser eingetaucht verbleibt, der Rasierzeit und der Zahl der Rasiervorgänge.

Es hat sich daher in der Praxis gezeigt, daß trotz der bekannten Gleitstreifen an Rasierapparaten mit wasserlöslichen Polymeren als Gleitmittel auf andere Rasierhilfen, wie Rasierschaum oder Rasiercreme nicht verzichtet werden kann, wie dies ursprünglich mit diesen Entwicklungen beabsichtigt war. Vielmehr ist zusammenfassend festzustellen, daß die bisherigen Versuche, durch Anwendung eines Rasierhilfsmittels die Naßrasur dahingehend zu vereinfachen, daß die bei der Rasur auftretenden Reibungskräfte reduziert werden, zwar teilweise erfolgreich waren, jedoch einige gravierende Nachteile mit sich brachten. Insbesondere die hohe Menge der Herauslösung an wasserlöslichem Polymer aus dem Gleitmittelträger im Verlaufe der Rasur erzeugt eine ungleichmäßige Wirkungsweise, die zu Beginn der Inbenutzungnahme einen übermässigen Schmiereffekt dergestalt erzeugt, daß sich auf der Haut des Benutzers ein antrocknender, schwer wieder abzuwaschender Film bildet, der ein klebriges Gefühl verursacht und die Gefahr von Hautirritationen nach sich ziehen kann, während am Ende der Lebensdauer nur noch außerordentlich wenig Gleitmittel abgegeben wird, so daß gerade zu dem Zeitpunkt, wo bereits durch Verschleiß die Schneidwirkung der Rasierklinge und damit der Rasierkomfort schlechter geworden ist, auch die zusätzliche Schmiermittelwirkung des Gleitstoffs nicht mehr ausreichend gegeben ist. Darüber hinaus ist das Verfahren zur Herstellung von Rasierklingeneinheiten bzw. Rasierapparaten mit solchen Gleitstreifen sehr kostenintensiv durch die komplizierte Fertigung entweder im Spritzgießverfahren von Kappe und Gleitstreifen getrennt mit anschließendem Zusammenfügen z.B. durch Kleben oder durch ein aufwendiges 2-Komponenten-Spritzgießverfahren sowie durch die hohen Kosten der als Gleitmittel verwendeten wasserlöslichen Polymere.

Der Erfindung liegt in Anbetracht dieses Standes der Technik die Aufgabe zugrunde, den bekannten Rasierapparat der eingangs genannten Art derart weiter zu entwickeln, daß die beim Rasieren aufzuwendende Oberflächenarbeit verringert wird, ohne daß die Nachteile auftreten, die ein Gleitmittel erzeugt, welches beim Rasiervorgang im Kontakt mit Wasser aus dem Trägermaterial herausgelöst wird und einen die Gleitfähigkeit verbessernden Schmierfilm auf der Haut des Benutzers bildet. Die Zielsetzung der Erfindung geht vielmehr dahin, den Rasierapparat mit einer im nassen Zustand hochgleitfähigen Oberfläche, insbesondere mit einem Reibungskoeffizienten zu entwickeln, der kleiner als der zwischen Haut und Metall ist,

2

wobei zu berücksichtigen ist, daß als Ergebnis durchgeführter Messungen feststeht, daß die Reibungskräfte zwischen den Partnern Polystyrol (übliches Material von Rasierapparaten)/Haut im nassen Zustand unter gleichen Testbedingungen erheblich über denen im trockenen Zustand liegen. Mit der Erfindung wird im Gegensatz zu diesen Ergebnissen gerade die gegenteilige Wirkung angestrebt, nämlich eine Verringerung der Reibungskräfte im nassen Zustand gegenüber denen im trockenen Zustand bei gleichen Partnern.

Die Aufgabe ist an einem Rasierapparat erfindungsgemäß dadurch gelöst, daß das Gleitmittel ein Xerogel ist, welches bei der Aufnahme von Wasser als Dispersionsmittel in ein auf der Haut des Benutzers hochgleitfähiges Lyogel mit einem Reibungskoeffizienten $\mu < 0,25$ übergeht. Mit der Erfindung ist ein prinzipiell vom Stand der Technik verschiedener Vorschlag zur Verringerung der Reibungskraft gemacht, da die Verwendung eines wasserlöslichen polymeren Filmbildners vermieden ist und anstelle dessen die beim Rasiervorgang die Haut des Benutzers berührende Fläche des Rasierapparats hochgleitfähig aufgrund stofftypischer Eigenschaften des Xerogels im nassen Zustand ist. Xerogele sind Gele, die ihre Flüssigkeit auf irgendeine Weise, z.B. durch Verdampfen, verloren haben. Die grundsätzliche Gelstruktur bleibt jedoch erhalten, die aus einem oder mehreren kolloidverteilten Stoff oder Stoffen mit langen oder stark verzweigten Teilchen und einer Flüssigkeit (meist Wasser) als Dispersionsmittel bestehen. Die kolloidverteilte Substanz bildet im Dispersionsmittel ein räumliches Netzwerk, wobei die Teilchen durch Neben- oder Hauptvalenzen an verschiedenen Punkten (Haftpunkte) aneinander haften. Sind die Zwischenräume zwischen den Teilchen mit Flüssigkeit ausgefüllt, so liegt ein Lyogel vor. Bei den durch Verdampfen hergestellten Xerogelen liegt ein Grenzzustand zum Festkörper vor. Durch Zugabe des Dispersionsmittels, beispielsweise von Wasser, gehen Xerogele durch Quellung wieder in Lyogele über. Die Oberfläche wird dabei schlüpfrig und weist einen niedrigen Reibungskoeffizienten auf. Bei diesem Vorgang geht die das Netzwerk bildende kolloide Substanz nicht in Lösung. Es bildet sich folglich beim Rasieren kein Film eines herausgelösten Gleitmittels auf der Haut, wie dies beim Stand der Technik der Fall ist.

Vorzugsweise besteht das Xerogel aus einem natürlichen Einstoffsystem, wie hochmolekularen Polysacchariden, oder einer homogenen Mischung aus Homopolymeren und Copolymeren von Vinylpyrrolidon, vorzugsweise Polyvinylpyrrolidon, sowie einem aromatischen Polyurethan als weiterer Komponente. Bei der Aufnahme des Dispersionsmittels Wasser während des Rasiervorgangs ergibt sich die hohe Gleitfähigkeit, wobei eine Herauslösung von Stoffen aufgrund der bei Gelen typischen intermolekularen Kräfte nicht zu befürchten ist.

Es ist zweckmäßig, bei einem aus Polyvinylpyrrolidon und dem Polyurethan bestehenden Xerogel den Anteil an Polyvinylpyrrolidon einzustellen auf ein Verhältnis PVP : PUR zwischen (3 - 5) : 1, d.h. es liegt ein 3-5facher Überschuß an PVP vor, nämlich 3,6 - 4 : 1. Dieses Verhältnis ist optimal bezüglich der Einstellung eines niedrigen Reibungskoeffizientens, dessen Wert über die Lebensdauer der Rasierklinge (normalerweise angesetzt auf sieben Tage) beibehalten wird, weiterhin bezüglich der Widerstandsfähigkeit gegen Verschleiß sowie der im Rahmen des Herstellungsverfahrens aufzuwendenden Trocknungszeit. Folgende Abhängigkeiten bestehen:

1. Der Reibungskoeffizient ist um so niedriger, je mehr Polyvinylpyrrolidon in dem Xerogel enthalten ist.

2. Je größer der Polyvinylpyrrolidon-Anteil im Xerogel ist, um so größer ist die Gefahr, daß die intermolekularen Bindungskräfte nicht ausreichen, das Polyvinylpyrrolidon in der Gelstruktur zu halten, so daß sich die Gefahr eines Herauslösens an Polyvinylpyrrolidon ergibt.

3. Die Widerstandsfähigkeit gegen Abrasion steigt mit dem Anteil an Polyurethan.

Große Vorteile werden erreicht, wenn das Polyurethan eine Dispersion mit Wasser und N-Methylpyrrolidon als Lösungsmittel ist, wobei vorzugsweise die Polyurethan-Dispersion einen Feststoffgehalt zwischen 30 und 33 % insbesondere 31 Gew. % aufweist und der Anteil an N-Methylpyrrolidon (NMP) zwischen 10 und 12 Gew %, insbesondere 11 Gew. %, Rest Wasser, beträgt. NMP ist ein hochsiedendes Lösungsmittel mit einer Siedetemperatur von ca. 204°C, was eine gewisse Verlängerung der Verdampfungszeit mitsichbringt, die wegen der hohen Umweltverträglichkeit jedoch in Kauf genommen wird. Es kann auf die üblichen organischen Lösungsmittel im wesentlichen verzichtet werden.

Gemäß einer bevorzugten Ausführungsform des erfindungsgemäßen Rasierapparats ist vorgeschlagen, das Xerogel als Beschichtung mit einer Schichtstärke zwischen 5 und 150 $\mu$m auf der Kappe und/oder der Führungsfläche des Rasierapparats auszubilden. Vorzugsweise wird eine Schichtstärke < 30 $\mu$m gewählt, da diese selbst in dem gequollenen Zustand des Lyogels ausreichende Stabilität besitzt. Bei zu großen Schichtstärken > 150 $\mu$m können Materialverluste durch abrasive Kräfte entstehen, da eine entsprechend hohe Wasseraufnahme (Quellvermögen) die Widerstandsfähigkeit gegenüber abrasiven Kräften verringert.

Die Beschichtung kann auf einer die Haut des Benutzers beim Rasiervorgang berührenden Fläche des Rasierapparats entweder direkt oder indirekt ausgebildet sein. Zur indirekten Ausbildung ist in weiterer Ausgestaltung der Erfindung vorgeschlagen, das Xerogel auf eine selbstklebende Trägerfolie anzuordnen und auf der entsprechenden Fläche des Rasierapparats anzukleben. Die Trägerfolie kann aus Polyvinylchlorid oder Polyester, vorzugsweise Polyäthylenterephthalat bestehen. Zur Verbesserung der Haftung ist es zweckmäßig, die beschichtete Trägerfolie entweder auf einem flachen Teil des Rasierapparats anzubringen oder auf einem gebogenen Teil mit einem Radius R > 20 mm. Damit wird eine ausreichende Haftung auf dem Rasierapparat gewährleistet und werden die auftretenden Reibungskräfte gleichmäßig über die gesamte Gleitfläche verteilt. Bei Anwendung einer Trägerfolie kann es vorteilhaft sein, diese in einer Ausnehmung, insbesondere der Kappe, anzuordnen, um eine ebene Berührungsfläche zu erzielen.

Mit der erfindungsgemäßen Xerogel-Schicht auf einem Rasierapparat ist eine kurze Aktivierungszeit durch das bei der Rasur vorhandene Wasser notwendig. Die Struktur des Beschichtungsmaterials ist so beschaffen, daß kleine Moleküle, wie Wasser, in das Gel-Netzwerk eindringen können und die Quellung des Xerogels binnen kurzer zeit bewirken können. Dies kann durch die Wahl des Matrixmaterials und die Weite des Netzwerks gesteuert werden. Im Gegensatz zu der bisher bekannten Verschlechterung der Gleiteigenschaften über die Lebensdauer, ergibt sich bei der Verwendung eines Xerogels als Gleitschicht mit der zunehmenden Anwendungszeit eine Verbesserung der Gleitwirkung, bedingt durch die Orientierung der Molekularketten an der Oberfläche. Die Haltbarkeit gegen ein Auslagen ist bei dem erfindungsgemäßen Rasierapparat erheblich verbessert.Selbst 24-stündiges Tauchen in Wasser verändert die Eigenschaften der Xerogel-Gleitschicht nicht. Auch eine Lagerung bei hoher Luftfeuchtigkeit (> 90 %) die bisher bekannte Gleitmittel unbrauchbar werden läßt, hat keinen Einfluß auf die Qualität des erfindungsgemäßen Rasierapparats. Schließlich ist auch die Temperaturstabilität des Xerogels sehr hoch, da sie im wesentlichen von der Temperaturstabilität des Trägermaterials bestimmt ist. Damit ergibt sich eine problemlose Handhabung für den Verbraucher bei gesicherten Gleiteigenschaften über die gesamte Anwendungszeit des Rasierapparats bzw. der Rasierklingeneinheit.

Durch die Erfindung wird ferner ein neues Verfahren zur Herstellung einer Gleitfläche an einem Rasierapparat vorgeschlagen, welches erfindungsgemäß dadurch gekennzeichnet ist, daß eine Lösung eines linearen, unvernetzten Polyurethans und eines wasserlöslichen Kunststoffes, insbesondere Polyvinylpyrrolidon, hergestellt wird, daß diese Lösung in einem Arbeitsgang als Schicht auf eine die Haut des Benutzers während des Rasiervorgangs berührende Fläche, insbesondere die Kappe des Rasierapparats, direkt oder indirekt aufgetragen wird und das Lösungsmittel bei erhöhter Temperatur zur Ausbildung eines Xerogels verdampft wird. Damit wird das gewünschte Xerogel direkt nach dem Verdampfen des Lösungsmittels in einem Schritt ohne zusätzliche Härtung erhalten, so daß der zeit-und verfahrenstechnische Aufwand zur Herstellung der Gleitschicht auf eine Minimum reduziert wird und somit die Anwendung für eine Massenprodukt Rasierapparat ermöglich ist.

Zur indirekten Auftragung ist es in zweckmäßiger Ausgestaltung der Erfindung vorgeschlagen, zunächst die Beschichtung einer selbstklebenden, ggf. mit einem Ätz- oder Polymerhaftvermittler vorbehandelten Trägerfolie aus Polyvinylchlorid oder Polyester, vorzugsweise Polyäthylenterephthalat, vorzunehmen und anschließend die Trägerfolie mit der gewünschten Oberfläche des Rasierapparats, insbesondere der Kappe, durch Kleben zu verbinden. Ebenso kann es bei der direkten Beschichtung der gewünschten Fläche des Rasierapparats zweckmäßig sein, eine Vorbehandlung mit einem Primer durchzuführen oder die Fläche zur Haftungsverbesserung 15 min bei erhöhter Temperatur, vorzugsweise 40° C, in einer Lösung aus Chromschwefelsäure vorzubehandeln. Der dabei verwendete Kunststoff ist ein ABS, das für galvanische Behandlung geeignet ist.

Im Rahmen des Herstellungsverfahrens ist es wichtig, eine homogene Mischung von Polyurethan und Polyvinylpyrrolidon herzustellen, wobei die Gewichtsverhältnisse Polyvinylpyrrolidon zu Polyurethan im Verhältnis zwischen 3 : 1 zu 5 : 1 liegen sollen und die Homogenität durch Rühren der Lösung direkt bis zum Auftragen zu gewährleisten. Der nach dem Auftragen durchzuführende Verdampfungsvorgang erfolgt zweckmäßigerweise bei einer Temperatur von etwa 70° C über eine Zeitdauer von weniger als 15 min.

Mit dem erfindungsgemäßen Verfahren ist eine für Massenprodukte anwendbare Auftragungstechnik entwickelt worden, mit der eine ausgezeichnete Verankerung des Xerogels auf dem Trägermaterial sichergestellt ist.

Gemäß einem Ausführungsbeispiel wird wie folgt verfahren:

Es wird zunächst ein Polyvinylpyrrolidon/Polyurethanblend gemäß nachfolgender Rezeptur hergestellt:

| Polyurethan-Dispersion | 81 g |
|---|---|
| Wasser | 250 g |
| Diacetonalkohol | 50 g |
| Polyvinylpyrrolidon K90 | 100 g |
| Isopropanol | 519 g |
| Fluorad FC430 | 1 g |

Die Kappe eines Rasierapparats wird in obiger Lösung getaucht und anschließend 10 min. lang einer Trocknung bei 70° C unterworfen. Eventuell kann es vorteilhaft sein, die Tauchlösung weiter zu verdünnen. Es bildet sich ein Xerogel aus, welches beim Rasieren Wasser als Dispersionsmittel aufnimmt und hochgleitfähige Oberflächeneigenschaften erhält.

In einem weiteren Ausführungsbeispiel wird folgende Mischung von Polyvinylpyrrolidon und Polyurethan verwendet:

| Polyurethan-Dispersion | 81 g |
|---|---|
| Wasser | 300 g |
| Diacetonalkohol | 60 g |
| Polyvinylpyrrolidon K90 | 90 g |
| Isopropanol | 469 g |
| Fluorad FC430 | 1 g |

Die Lösung dieser Stoffe wurde auf einen Polyvinylchloridfilm einer Stärke von 150 $\mu$m aufgetragen und innerhalb von 10 min bei 75° C das Lösungsmittel verdampft. Der derart beschichtete PVG-Film wurde in Streifen geschnitten. Ein Streifen wurde durch Kleben mit der Kappe eines Rasierapparats verbunden. Als Klebstoff wurde wasserfestes Polyacrylat in einer Stärke von 10 bis 15 $\mu$m verwendet.

Weitere Einzelheiten, Merkmale und Vorteile des Gegenstandes der Erfindung ergeben sich aus der nachfolgenden Beschreibung der zugehörigen Zeichnung. In der Zeichnung zeigt

Fig. 1    eine Rasierklingeneinheit, teilweise im Schnitt,

Fig. 2    einen einzelnen Gleitstreifen zur Verwendung an einer Rasierklingeneinheit gemäß Fig. 1 im Schnitt und

Fig. 3    den Gleitstreifen in einer Draufsicht.

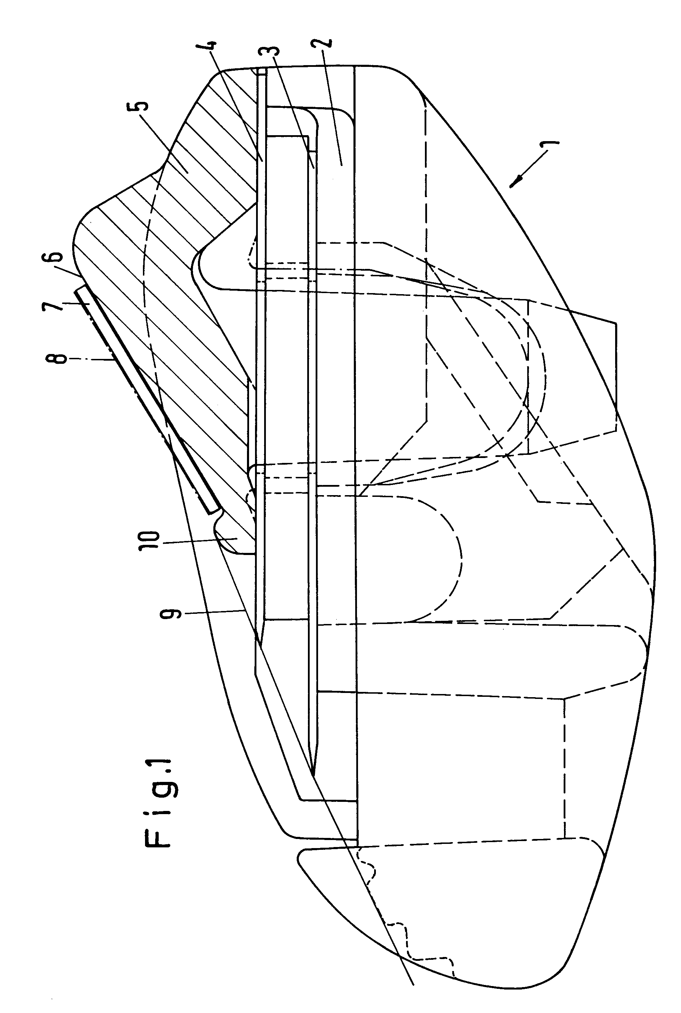

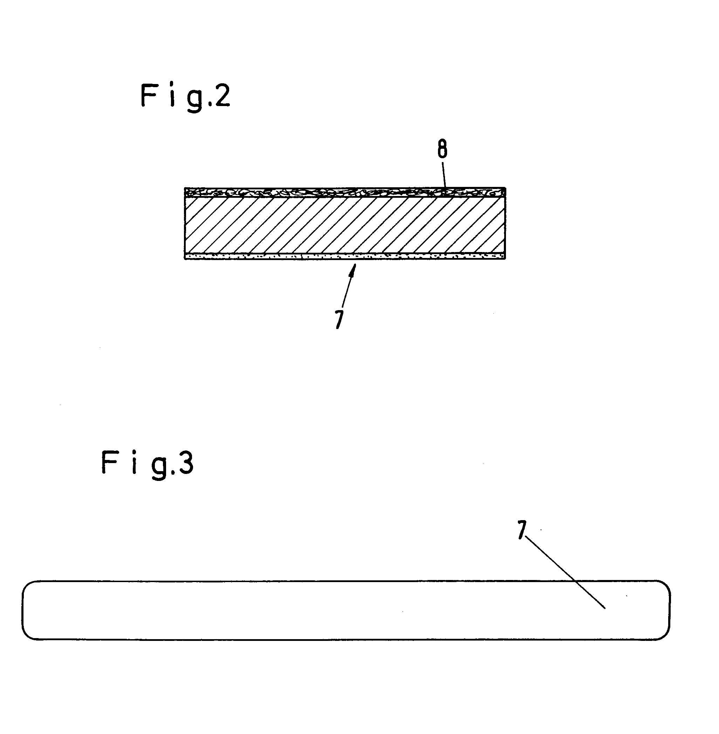

Die Rasierklingeneinheit 1 in Fig. 1 der Zeichnung wird in an sich bekannter Weise mit einem mit passenden Verbindungsmitteln ausgerüsteten Rasierapparategriff verbunden und bildet dieserart den vom Benutzer zu gebrauchenden Rasierapparat. Er weist einen Träger 2 für zwei Rasierklingen 3, 4 und eine Kappe 5 auf. Auf der Vorderseite der Kappe 5 ist eine ebene Fläche 6 ausgebildet, die zur Aufnahme eines Gleitstreifens 7 geeignet ist. Die ebene Fläche 6 endet vorn in einer abgerundeten Schutzleiste 10, die einerseits dazu dient, die Positionierung des Gleitstreifens 7 beim Zusammenbau zu erleichtern und andererseits dessen Vorderkante beim Rasiervorgang schützt. Dessen Aufbau ist den Fig. 2 und 3 der Zeichnung entnehmbar. Der Gleitstreifen 7 weist eine vorderseitige Gleitfläche 8 aus einem Xerogel auf, welcher in bezug zum frontseitigen Ende der Kappe 5 so angeordnet ist, daß ein ebener Übergang von einer gedachten die Schneidkanten der beiden Rasierklingen tangierenden Verbindungslinie über das frontseitige Ende der Kappe 5 zur Gleitfläche 8 im Sinne der auszuführenden Rasierbewegung geschaffen ist. Zu der geometrischen gedachten Verbindungslinie 9 der Schneidkanten der beiden Rasierklingen ist die Gleitfläche 8 etwas schräg nach vorn angestellt, um einen erhöhten Hautkontakt im Benutzungsfall sicherzustellen.

Gemäß Fig. 2 der Zeichnung ist der Gleitstreifen 7 aus mehreren Schichten aufgebaut. Die mittlere Schicht ist eine Polyvinylchlorid-Trägerfolie einer Stärke von 150 $\mu$m ± 10 % deren Unterseite mit einem Polyacrylat-Klebstoff beschichtet ist. Auf der Oberseite ist ein Coating aus einem Polyvinylpyrrolidon/Polyurethan-Blend gemäß obigen Rezepturen als Xerogel aufgebracht. Die Schicht weist eine Stärke von 25 $\mu$m ± 5 auf.

Ein derartiger Gleitstreifen 7 mit der Gleitfläche 8 wird auf einer Rasierklingeneinheit 1 mit der wasser festen Polyacrylat-Klebeschicht, die 10 bis 15 $\mu$m dick ist, aufgeklebt, wobei zur Verbesserung dieses Vorgangs gemäß Fig. 3 abgerundete Ecken vorgesehen sind. Im Falle einer Benutzung des Rasierapparats bildet sich durch Wasseraufnahme sehr spontan aus dem Xerogel ein Lyogel mit ausgezeichneten Gleiteigenschaften, die die beim Rasieren aufzuwendende Oberflächenarbeit wesentlich verringern. Aufgrund der Herabsetzung des Reibungswiderstands wird eine komfortablere und effektivere Rasur erzielt. Ein nennenswerter Verschleiß der Gleitfläche 8 durch Abrasion ist über die übliche Lebensdauer der Rasierklingen 3, 4 nicht festzustellen.

**Patentansprüche**

1. Rasierapparat bzw. Rasierklingeneinheit mit einem Träger (2) für mindestens eine Rasierklinge (3, 4), bei dem eine die Haut des Benutzers während des Rasiervorgangs berührende Fläche (8) mit einem Gleitmittel (7) versehen ist,
**dadurch gekennzeichnet**,
daß das Gleitmittel (7) ein Xerogel ist, welches bei der Aufnahme von Wasser als Dispersionsmittel in ein auf der Haut des Benutzers hochgleitfähiges Lyogel mit einem Reibungskoeffizienten $\mu < 0,25$ übergeht.

2. Rasierapparat nach Anspruch 1, dadurch gekennzeichnet, daß das Xerogel aus einem natürlichen Einstoffsystem, insbesondere hochmolekularen Polysacchariden, oder einer homogenen Mischung aus Homopolymeren und Copolymeren von Vinylpyrrolidon, vorzugsweise Polyvinylpyrrolidon, sowie einem Polyurethan als weiterer Komponente besteht.

3. Rasierapparat nach Anspruch 1 und 2, dadurch gekennzeichnet, daß das Polyurethan ein aromatisches Polyurethan ist.

4. Rasierapparat nach einem der Ansprüche 2 oder 3, dadurch gekennzeichnet, daß bei einem aus Polyvinylpyrrolidon und dem Polyurethan bestehenden Xerogel der Anteil an Polyvinylpyrrolidon zu Polyurethan auf ein Verhältnis PVP : PUR zwischen (3 - 5) : 1 eingestellt ist.

5. Rasierapparat nach Anspruch 4, dadurch gekennzeichnet, daß das Verhältnis PVP : PUR 3,6 - 4 : 1 beträgt.

6. Rasierapparat nach einem der Ansprüche 2 bis 5, dadurch gekennzeichnet, daß das Polyurethan eine Dispersion mit Wasser und N-Methylpyrrolidon als Lösungsmittel ist, wobei vorzugsweise die Polyurethan-Dispersion einen Feststoffgehalt zwischen 30 und 33 % insbesondere 31 Gew. % aufweist und der Anteil an N-Methylpyrrolidon zwischen 10 und 12 Gew. % insbesondere 11 Gew. %, Rest Wasser, beträgt.

7. Rasierapparat nach einem der Ansprüche 1 bis 6, dadurch gekennzeichnet, daß das Xerogel mit einer Schichtstärke zwischen 5 $\mu$m und 160 $\mu$m auf einer Kappe des Rasierapparates und/oder einer Führungsfläche des Rasierapparates direkt oder indirekt ausgebildet ist.

8. Rasierapparat nach Anspruch 7, dadurch gekennzeichnet, daß eine Schichtstärke < 30 $\mu$m eingestellt ist.

9. Rasierapparat nach einem der Ansprüche 1 bis 8, dadurch gekennzeichnet, daß das Xerogel auf einer selbstklebenden Trägerfolie angeordnet ist.

10. Rasierapparat nach Anspruch 9, dadurch gekennzeichnet, daß die Trägerfolie aus Polyvinylchlorid oder Polyester, vorzugsweise Polyäthylenterephthalat, besteht.

11. Rasierapparat nach Anspruch 9 und 10, dadurch gekennzeichnet, daß die mit Xerogel beschichtete Trägerfolie entweder auf einem flachen Teil des Rasierapparats oder auf einem gebogenen Teil mit einem Radius R > 20 mm angeordnet ist.

12. Rasierapparat nach einem der Ansprüche 9 bis 11, dadurch gekennzeichnet, daß die Trägerfolie in einer Ausnehmung, insbesondere der Kappe (5) des Rasierapparats, angeordnet ist.

13. Rasierapparat nach Anspruch 12, dadurch gekennzeichnet, daß die Ausnehmung als ebene Fläche gestaltet ist, die vorn in einer abgerundeten etwa in Gleitstreifenstärke hochstehenden Schutzleiste (10) endet.

**14.** Rasierapparat nach Anspruch 12 und 13, dadurch gekennzeichnet, daß die Trägerfolie bzw. der Gleitstreifen (7) schräg nach vorn geneigt aus der Rasierebene angestellt ist, um einen erhöhten Hautkontakt zu bewirken.

**15.** Verfahren zur Herstellung der Gleitfläche an einem Rasierapparat gemäß einem der Ansprüche 1 bis 12,
**dadurch gekennzeichnet,**
daß eine Lösung eines linearen, unvernetzten Polyurethans und eines wasserlöslichen Kunststoffs, insbesondere Polyvinylpyrrolidon, hergestellt wird, daß diese Lösung in einem Arbeitsgang als Schicht auf eine die Haut des Benutzers während des Rasiervorgangs berührende Fläche, insbesondere die Kappe (5) des Rasierapparats, direkt oder indirekt aufgetragen wird und das Lösungsmittel bei erhöhter Temperatur zur Ausbildung eines Xerogels verdampft wird.

**16.** Verfahren nach Anspruch 15, dadurch gekennzeichnet, daß zur indirekten Austragung zunächst die Beschichtung einer selbstklebenden, ggf. mit einem Ätz- oder Polymerhaftvermittler vorbehandelten Trägerfolie aus Polyvinylchlorid oder Polyester, vorzugsweise Polyäthylenterephthalat, vorgenommen wird und anschließend die Trägerfolie mit der gewünschten Fläche des Rasierapparats, insbesondere der Kappe (5), durch Kleben verbunden wird.

**17.** Verfahren nach Anspruch 15, dadurch gekennzeichnet, daß bei der direkten Beschichtung eine Vorbehandlung der zu beschichtenden Fläche des Rasierapparats mit einem Primer durchgeführt wird oder daß die Fläche zur Haftverbesserung 15 min bei erhöhter Temperatur, vorzugsweise 40° C, in einer Lösung aus Chromschwefelsäure vorbehandelt wird.

**18.** Verfahren nach einem der Ansprüche 15 bis 17, dadurch gekennzeichnet, daß eine homogene Mischung von Polyurethan und Polyvinylpyrrolidon in Form einer Lösung hergestellt wird, wobei die Gewichtsverhältnisse Polyvinylpyrrolidon zu Polyurethan im Verhältnis zwischen 3 : 1 und 5 : 1 liegen und die Homogenität durch ständiges Rühren gewährleistet ist, und daß der nach dem Auftragen durchzuführende Verdampfungsvorgang bei einer Temperatur von > 70° C über eine Zeitdauer von weniger als 15 min durchgeführt wird.

**Claims**

**1.** Safety razor and/or razor blade unit with a platform (2), for at least one razor blade (3, 4), where a surface (8) in contact with the skin of the user during the shaving process is provided with a lubricant (7),
characterized in
that the lubricant (7) is a xerogel, which on the absorption of water as a dispersion medium becomes a lyogel with a coefficient of friction $\mu < 0.25$ and is highly slidable on the user's skin.

**2.** Safety razor as in claim 1, characterized in that the xerogel consists of a natural unary system, in particular polysaccharides of high molecular weight, or a homogeneous mixture of homopolymers and copolymers of vinyl pyrrolidone, preferably polyvinyl pyrrolidone, as well as a polyurethane as a further component.

**3.** Safety razor as in claims 1 and 2, characterized in that the polyurethane is an aromatic polyurethane.

**4.** Safety razor as in one of claims 2 or 3, characterized in that in a xerogel consisting of polyvinyl pyrrolidone and the polyurethane the proportion of polyvinyl pyrrolidone to polyurethane is set at a ratio of PVP:PUR between (3-5):1.

**5.** Safety razor as in claim 4, characterized in that the PVP:PUR ratio is 3.6-4:1.

**6.** Safety razor as in one of claims 2 to 5, characterized in that the polyurethane is a dispersion with water and N-methylpyrrolidone as solvent, where preferably the polyurethane dispersion has a solids content between 30 and 33%, In particular 31% w/w and the proportion of N-methyl pyrrolidone is between 10 and 12% w/w, in particular 11% w/w, with the rest water.

**7.** Safety razor as in one of claims 1 to 6, characterized in that the xerogel is directly or indirectly formed on a cap and/or a guard of the surface of the razor with a coating thickness between 5 $\mu$m and 160 $\mu$m.

**8.** Safety razor as in claim 7, characterised in that a coating thickness of < 30 $\mu$m is set.

**9.** Safety razor as in one of claims 1 to 8, characterized in that the xerogel is arranged on a self-adhesive carrier film.

**10.** Safety razor as in claim 9, characterized in that the carrier film consists of polyvinyl chloride or polyester, preferably polyethylene phthalate.

**11.** Safety razor as in claims 9 and 10, characterized in that the carrier film is coated with xerogel and is arranged either on a flat part of the safety razor or on a curved part with a radius R > 20 mm.

**12.** Safety razor as in one of claims 9 to 11, characterized in that the carrier film is arranged in a particular on the cap of the safety razor.

**13.** Safety razor as in claim 12, characterized in that the recess is shaped as a flat surface which ends in front in a rounded guardbar (10) raised about the thickness of the sliding strip.

**14.** Safety razor as in claims 12 and 13, characterized in that the carrier film and the sliding strip (7) is set angled forward from the plane of shaving in order to give increased skin contact.

**15.** Process for making the sliding surface on a safety razor as in any one of claims 1 to 12, characterized in that
a solution of a linear, unlinked polyurethane and a water-soluble plastic, in particular polyvinyl pyrrolidone, is made, that this solution is applied directly or indirectly in one operation as a coating to a surface, in particular cap (5) of the safety razor, in contact with the skin of the user during the shaving process and solvent is evaporated at raised temperature to form a xerogel.

**16.** Process as in claim 15, characterized in that for indirect application a self-adhesive carrier film of polyvinyl chloride or polyester, preferably polyethylene phthalate, possibly pretreated with a caustic or polymer bonding aid, is coated and then the carrier film is joined to a desired surface of the safety razor, in particular the cap (5) by bonding.

**17.** Process as in claim 15, characterized in that with direct coating the surface of the safety razor to be coated is pretreated with a primer or that to improve adhesion the surface is pretreated for 15 min at a raised temperature, preferably 40°C, in a solution of chromatisulphuric acid.

**18.** Process as in one of claims 15 to 17, characterised in that a homogeneous mixture of polyurethane and polyvinyl pyrrolidone is made in the form of a solution, the ratio by weight of polyvinyl pyrrolidone to polyurethane between 3:1 and 5:1 and the homogeneity is ensured by constant stirring and that the evaporation process to be carried out after application is carried out at a temperature of > 70°C for a time of less than 15 min.

**Revendications**

**1.** Rasoir ou unité à lames de rasoir, qui comporte un support (2) pour au moins une lame de rasoir (3, 4), et sur lequel une surface (8) entrant en contact avec la peau de l'utilisateur au cours du rasage, est pourvue d'un agent antifriction (7),
caractérisé en ce que
l'agent antifriction (7) est un gel xérophile, qui lors de l'absorption d'eau en tant qu'agent de dispersion, se transforme en un gel lyophile présentant une très grande aptitude au glissement sur la peau de l'utilisateur, et un coefficient de frottement $\mu$ < 0,25.

**2.** Rasoir selon la revendication 1, caractérisé en ce que le gel xérophile est formé par un constituant unique naturel, tel que des polysaccharides de poids moléculaire élevé, ou par un mélange homogène d'homopolymères ou de copolymères de vinylpyrrolidone, de préférence polyvinylpyrrolidone, ainsi qu'un polyuréthanne en tant qu'autre constituant.

3. Rasoir selon les revendications 1 et 2, caractérisé en ce que le polyuréthanne est un polyuréthanne aromatique.

4. Rasoir selon l'une des revendications 2 ou 3, caractérisé en ce que dans le cas d'un gel xérophile constitué de polyvinypyrrolidone et du polyuréthanne, la proportion de polyvinypyrrolidone par rapport au polyuréthanne est établie de telle sorte que le rapport PVP : PUR soit compris entre (3 - 5) : 1.

5. Rasoir selon la revendication 4, caractérisé en ce que le rapport PVP : PUR prend une valeur de 3,6 - 4 : 1.

6. Rasoir selon l'une des revendications 2 à 5, caractérisé en ce que le polyuréthanne est une dispersion avec de l'eau et N-méthylpyrrolidone comme solvant, la dispersion de polyuréthanne présentant de préférence une teneur en substance solide comprise entre 30 et 33%, de préférence 31% en poids, et la part de N-méthylpyrrolidone étant comprise entre 10 et 12% en poids, de préférence 11% en poids, et le reste étant de l'eau.

7. Rasoir selon l'une des revendications 1 à 6, caractérisé en ce que le gel xérophile est formé directement ou indirectement, en présentant une épaisseur de couche comprise entre 5 $\mu$m et 160 $\mu$m, sur un cabochon du rasoir et/ou une surface de guidage du rasoir.

8. Rasoir selon la revendication 7, caractérisé en ce que l'on établit une épaisseur de couche < 30 $\mu$m.

9. Rasoir selon l'une des revendications 1 à 8, caractérisé en ce que le gel xérophile est disposé sur une feuille autocollante formant substrat.

10. Rasoir selon la revendication 9, caractérisé en ce la feuille formant substrat est réalisée en chlorure de polyvinyle ou en polyester, de préférence en polyéthylène-téréphtalate.

11. Rasoir selon les revendications 9 et 10, caractérisé en ce que la feuille formant substrat revêtue, est rapportée, soit sur une partie plane du rasoir, soit sur une partie courbe avec un rayon R > 20 mm.

12. Rasoir selon l'une des revendications 9 à 11, caractérisé en ce que la feuille formant substrat est disposée dans un évidement, notamment du cabochon du rasoir.

13. Rasoir selon la revendication 12, caractérisé en ce que l'évidement se présente sous la forme d'une surface plane qui se termine à l'avant par une barrette de protection (10) arrondie et en saillie d'une valeur correspondant sensiblement à l'épaisseur de la bande antifriction.

14. Rasoir selon les revendications 12 et 13, caractérisé en ce que la feuille formant substrat, à savoir la bande antifriction (7), présente une incidence par rapport au plan de rasage en étant inclinée vers l'avant, en vue d'assurer un meilleur contact avec la peau.

15. Procédé de fabrication d'une surface antifriction sur un rasoir selon l'une des revendications 1 à 12, caractérisé
en ce que l'on fabrique une solution d'un polyuréthanne linéaire non réticulé et d'une matière plastique soluble dans l'eau, notamment du polyvinylpyrrolidone, en ce que cette solution est appliquée directement ou indirectement, en une seule opération et sous forme de couche, sur une surface du rasoir entrant en contact avec la peau de l'utilisateur au cours du rasage, notamment le cabochon du rasoir, et en ce que le solvant est évaporé à une température plus élevée en vue de former un gel xérophile.

16. Procédé selon la revendication 15, caractérisé en ce que pour l'application indirecte, l'on entreprend tout d'abord le revêtement d'une feuille autocollante formant substrat, le cas échéant traitée au préalable par un agent de décapage ou d'accrochage de polymère, en chlorure de polyvinyle ou en polyester, de préférence en polyéthylène-téréphtalate, et l'on relie ensuite, par collage, la feuille formant substrat à la surface souhaitée du rasoir, notamment du cabochon (5).

17. Procédé selon la revendication 15, caractérisé en ce que dans le cas du revêtement direct, l'on effectue un traitement préalable de la surface souhaitée du rasoir, par une couche de fond, ou bien en ce que l'on traite au préalable la surface pour améliorer l'accrochage, à une température plus élevée, de préférence 40°C, dans une solution sulfo-chromique.

18. Procédé selon l'une des revendications 15 à 17, caractérisé en ce que l'on fabrique un mélange homogène de polyuréthanne et de polyvinylpyrrolidone sous la forme d'une solution, la proportion en poids de polyvinylpyrrolidone par rapport au polyuréthanne étant dans un rapport compris entre 3 : 1 et 5 : 1, et l'homogénéité étant assurée par une agitation constante, et en ce que l'opération d'évaporation devant être effectuée après l'application, se fait à une température > 70°C, pendant une durée de moins de 15 min.

Fig.1

EP 0 321 679 B1

Fig.2

Fig.3